# EUROPEAN PATENT SPECIFICATION

(11) **EP 2 697 520 B1**
(45) Date of publication and mention of the grant of the patent: **04.04.2018**
(21) Application number: 12771947.4
(22) Date of filing: 11.04.2012
(51) Int. Cl.: A23P 10/30, A23L 29/10, A23L 21/25

(54) **CAPSULES COMPRISING AN EMULSIFIED SYRUP AND METHODS OF MAKING THE SAME**
KAPSELN MIT EINEM EMULGIERTEN SIRUP UND HERSTELLUNGSVERFAHREN DAFÜR
CAPSULES COMPRENANT UN SIROP ÉMULSIFIÉ ET LEURS PROCÉDÉS DE FABRICATION

(30) Priority: 12.04.2011 AU 2011901374
(43) Date of publication of application: 19.02.2014
(73) Proprietor: R.P. Scherer Technologies, LLC, Las Vegas, Nevada 89119 (US)
(72) Inventor: LIN, Jing, Keysborough, Vic 3173 (AU)
(74) Representative: Dunleavy, Kevin James
(86) International application number: PCT/US2012/033091
(87) International publication number: WO 2012/142137

(56) References cited:
- KR-A- 20090 116 894
- US-A- 4 276 312
- US-A- 4 804 542
- US-A- 5 569 466
- US-A1- 2004 126 463

## Description

### Field of the Invention

This invention relates to capsules and, more specifically to soft capsules, wherein the fill material comprises an emulsified syrup. In particular, the present invention relates to means of encapsulating honey in a gelatin, modified-gelatin or gelatin-free shell material. Furthermore, the capsules described herein are capable of being manufactured using a rotary die apparatus.

### Background of the Invention

Syrups, such as honey, maple syrup, molasses, sugar cane syrup, malt extract and other viscous syrups; principally containing sucrose, glucose, fructose and/or other sugars dissolved in water; find wide application as industrial food ingredients, as well as in cooking, beverages, and as spreads. Of all the syrups, honey has a particularly universal appeal. Indeed, honey is one of the oldest foods known to man, consumed both for its palatability and its nutritive value.

Furthermore, honey has been used by humans for centuries to treat a variety of ailments via topical application. More recently, its antiseptic and antibacterial properties have been chemically explained.

KR 2009 0116894 A exemplifys soft gelatin capsule formulations containing 5-50 weight % of concentrate of ginseng or red ginseng, 1-10 weight % of cyclodextrine, honey, fructose, sorbitol, glycerin, stevioside, or squalene. The capsules are coated with a coating that comprises 30-60 weight % of gelatin, 20-50 weight % of glycerin and D-sorbitol liquid, 5-30 weight % of D-mannitol, and 5-20 weight % of purified water.

U.S. Patent no. 4,276,312 A is directed to capsules filled with spray dried compositions. Example 1 of this document employs Maltrin-10, butylated hydroxyaninsole, polyoxyethylenesorbitan monooeleate (an emulsifier) and lemon oil.

U.S. Patent no. 5,569,466A discloses fill compositions for soft elastic capsules. Example 7 of this document is a composition of lycasin (hydrogenated glucose syrup), peppermint oil and polysorbate 80 (an emulsifier).

US 2004/0126463 A1 discloses a capsule filled with honey for use as a dietary supplement.

Syrups referred to herein are characterized by high viscosity, stickiness, great sweetness, high density, high water content, and hygroscopicity.

Fresh honey is a supersaturated liquid, containing more sugar than the given water content can typically dissolve at ambient temperatures, resulting in a density of approximately 1.36 grams per milliliter. In addition to the syrup characteristics above, honey is also considered a supercooled liquid and enjoys relative immunity from spoilage.

Three of the critical problems attributed to the use of honey, in each of industrial, medicinal and individual capacities, are its high viscosity and associated handling problems; its high water content; as well as its natural affinity to return to a crystallized state.

The present invention provides a new dosage form for syrups, in particular for honey, whereby a given syrup composition is encapsulated, preferably in gelatin-free soft shell capsules, ultimately simplifying the handling. Given the intrinsic nutritional and medicinal benefits of honey, as well as its fast absorption in the human body, the new delivery system described herein, may result in subsequent enhanced bioavailability for pharmaceuticals and particularly health and nutritional supplements formulated therewith.

Soft capsules, and most commonly, soft gelatin capsules; offer significant advantages over other dosage forms. They are more readily accepted by patients, since the capsules are easy to swallow and, in some instances, may not need to be flavoured in order to mask the unpleasant taste of the active agent.

Soft capsules are also more easily transported by patients than bulk liquids, since only the required number of doses need be removed from the package.

Soft capsule encapsulation of drugs further provides the potential to improve the bioavailability of pharmaceutical agents. Active ingredients are rapidly released in liquid form as soon as the shell ruptures. Complete disintegration of the capsule is not necessary for the active ingredients to become available for absorption, unlike the case of tableted compositions. Also, relatively insoluble active ingredients can be dispersed in a liquid carrier to provide faster absorption.

Attempts to encapsulate honey in capsules, particularly soft forms of capsules, had been previously unsuccessful. Its high density and water content often resulted in seam sagging, serious leaking and subsequent breakage of the resulting capsules.

In addition to problems resulting from the high density and water content, the high viscosity of honey also presents significant challenges in the manufacturing process. Conventional technologies rely on medicine pumps to pump the fill composition. However, in light of the high viscosity, pumps often seize or block. Similarly, transfer tubes often burst, and/or produce an uneven fill volume.

Moreover, the high viscosity cannot be immediately solved by the application of heat alone. Use of heat to reduce viscosity of liquid fills is well known in the art. However, exposing honey to elevated temperatures results in deterioration of flavour, aroma and colour. Additionally, the higher the temperature and the longer the exposure, the more severe the quality deterioration. Temperatures in excess of about 60°C can be deleterious. Not only does heat reduce the quality of the fill composition, application of heat also results in expanded fill volume which ultimately reduces the sealing strength of the resulting capsule. A reduction in sealing strength, results in leaking capsule seams and ultimately, an unviable product.

Furthermore, the natural crystallization of syrups, particularly honey, often results in a localized increase in the water content in portions of the honey solution that do not form part of the crystallised lattice. This localized increase in water content renders the honey more susceptible to fermentation, which is extremely undesirable.

Additionally, attempts to encapsulate honey in conventional gelatin soft capsules, results in shell hardening and crosslinking over time, due to the interaction between gelatin and the sugars in honey. Such an effect significantly reduces the shelf-life, viability, stability and quality of the product.

These intrinsic difficulties in encapsulating honey, in either of soft or hard capsules, are further evidenced by the absence of equivalent products in the marketplace. As such, the present state of the art fails to address these inherent problems.

In order to overcome the inherent difficulties in encapsulating honey, the Applicant was required to address:
- the handling and manufacturing of a highly viscous product;
- the stability and viability of a high water content product;
- the tendency to crystallize resulting in a non-uniform product;
- the limited ability to heat the desired fill product;
- the need to maintain an excellent flavour profile faithful to the natural substance; and
- the incompatibility of conventional gelatin-based softgels with unmodified syrup.

The discussion of documents, acts, materials, devices, articles and the like is included in this specification solely for the purpose of providing a context for the present invention. It is not suggested or represented that any or all of these matters formed part of the prior art base or were common general knowledge in the field relevant to the present invention as it existed before the priority date of each claim of this application.

Where the terms "comprise", "comprises", "comprised" or "comprising" are used in this specification (including the claims) they are to be interpreted as specifying the presence of the stated features, integers, steps or components, but not precluding the presence of one or more other features, integers, steps or components, or groups thereof.

The term "syrup", as used herein, refers to a viscous liquid comprising sugar and water; characterized by high viscosity, stickiness, great sweetness, high density, high water content, and hygroscopicity. Examples of suitable syrups include, but are not limited to, honey, maple syrup, molasses, sugar cane syrup and malt extract.

The term "capsule", as used herein, refers to a case or shell having a chamber; said chamber encapsulating a desired fill material, particularly a complementary health or pharmaceutical product. In one preferred embodiment said capsule is soluble or semi-soluble.

The term "honey", as used herein, is inclusive of liquid honey, pasteurized honey, raw honey, strained honey, ultrafiltered honey, ultrasonicated honey, creamed honey, whipped honey, clarified honey, non-clarified honey and crystallized honey.

### Summary of Invention

The present invention provides a capsule for a complementary health or pharmaceutical product comprising:
(a) A hard or soft shell for encapsulating a fill material; and
(b) A fill material comprising an emulsified syrup wherein the emulsified syrup comprises i) 40 to 50% wt/wt of at least one syrup that includes a component selected from liquid honey, creamed honey and crystallized honey, (ii) 1 to 10% wt/wt of at least one emulsifier; and (iii) 40 to 55% wt/wt of at least one oil or oil mixture; and (iv) 1 to 5% wt/ wt of water.
The emulsified syrup comprises at least one syrup, at least one emulsifier and at least one oil or oil mixture, and water. More preferably the syrup is liquid honey or crystallized honey.

In another preferred embodiment, the fill material further comprises one or more complementary health and/or pharmaceutically active compounds.

In a further preferred embodiment, the shell material of the present invention may comprise a gelatin free material. A capsule manufactured from a preferred gelatin free shell material, has been previously patented by the Applicant under Australian Patent No. 735699 and Australian Innovation Patent No. 2006100743. As such, in one preferred embodiment the shell material comprises a mixture of iota-carrageenan and at least one modified starch; water, a plasticizer and a buffer.

In another preferred embodiment, the present invention provides a process for producing a capsule for a complementary or pharmaceutical product as disclosed in claim 14. The formulation and the process described herein, enables the manufacturing of soft capsules containing high water, high sugar content, high density and high viscosity raw materials in fills, such as syrups. The capsules are of good appearance after overcoming seam sagging and serious leaking problems.

The principle of the formulation is by emulsifying the syrup, for example honey, in an oil or oil mixture so that the viscosity, density and water content are reduced to the extent that manufacturing with a rotary die process is feasible.

Given the intrinsic nutritional benefit of honey and its fast absorption in the human body, the applications of the capsules described herein may be extended to a new delivery system for enhanced bioavailability for pharmaceuticals and particularly health and nutritional supplements.

### Description of the Invention

The encapsulation of syrups, particularly honey, in a single dosage form capsules offers significant advantages not only in handling and accessibility, but also in the provision of new delivery systems for pharmaceuticals and other complementary health supplements.

Conventional gelatin-based capsules are preferred in the art. However, as previously described, the gelatin is incompatible with an unmodified honey fill composition.

The formulation and associated process described herein, provides a means for encapsulating honey, using an emulsified syrup, specifically an emulsified honey. The honey includes liquid or creamed honey. Ideally, it should be free of crystals. However, the methods and formulation described herein are compatible with crystal sizes up to 180 µm. An example is creamed honey.

The present invention provides a capsule for a complementary health or pharmaceutical product comprising:
(a) A hard or soft shell for encapsulating a fill material; and
(b) A fill material comprising at least one emulsified syrup wherein the emulsified syrup comprises 40 to 50% wt/wt of at least one syrup selected from liquid honey, creamed honey and crystallized honey, (ii) 1 to 10% wt/wt of at least one emulsifier; and (iii) 40 to 55% wt/wt of at least one oil or oil mixture; and (iv) 1 to 5% wt/wt of water.
Specifically, the capsules of the invention constitute a single dose; a wider variation in capsule sizes is available compared to conventional capsules, and capsules can be manufactured to suit a given dosage requirement.

The Applicant recognized that a syrup, in particular for example pure honey; with a high water and sugar content, high viscosity and high density; was not in itself suitable for convention capsule manufacture. Where encapsulation of pure honey was attempted, the resulting capsules were soft and fragile. It was difficult for the shell to carry a fill material of such a high density through the encapsulation and drying process without subsequent breaking, leaking or deformation.

Furthermore, the encapsulation apparatus, in particular, the associated pumps were compromised when attempting encapsulation of pure honey. Transfer tubes often burst due to the high viscosity, or processing this high viscosity fill liquid resulted in an uneven fill volume and dose content dis-uniformity. Application of heat, to reduce the viscosity, resulted in an expanded fill volume, which subsequently reduced the sealing strength, and caused additional leakage. Finally the high water content, caused attempted pure honey capsules to deform after drying, and thus, such capsules were not presentable as a commercial product.

To enable the encapsulation of a syrup or syrup composition, for example honey, required a specific formulation technique, in particularly emulsification of said syrup, to address the inherent problems associated with viscosity, high water content and high sugar content. Furthermore, the formulation and process of the present invention enables honey to be encapsulated in a continuous manner with a rotary die process on a commercial scale. As such, the emulsified syrup of the present invention comprises at least one syrup, at least one emulsifier and at least one oil or oil mixture, and optionally water. More preferably the syrup is liquid honey, creamed honey or crystallized honey.

The emulsifier comprises at least one ionic or a non-ionic edible surfactant, preferably selected from the group comprising lecithin, polyoxyethylene fatty acid esters, glycerides of fatty acids, polyoxyethylene castor oil derivatives and sorbitan esters.

The oil or oil mixture preferably comprises at least one medium or long chain, mono-, di- or tri-glycerides; preferably selected from the group consisting of soya oil, sunflower oil, safflower oil and rice bran oil. The total water content of the fill material is preferably less than 15% wt/wt; and more preferably less than 12.5% wt/wt.

The final composition of the fill material of the present invention enables honey to be encapsulated in a continuous manner with a rotary die process on a commercial scale. As such, the fill material is formulated to provide a composition with an appropriate viscosity, water content, sugar content and uniformity, whilst maintaining the palatability and taste profile of the pure syrup or syrup mixture from which it is derived. The fill material of the present invention comprises syrup in the range of 40 to 50% wt/wt; emulsifier in the range of 1 to 10% wt/wt; oil or oil mixture in the range of 40 to 55% wt/wt; and water in the range of 1 to 5% wt/wt.

In another preferred embodiment, the shell material and/or fill material further comprises one or more pharmaceutically active compounds. In addition to its well known nutritional value, honey offers significant medicinal benefits, including antibacterial and antiseptic activity. Furthermore, an emulsified honey composition has the capacity to dissolve or suspend actives in either hydrophilic or hydrophobic phases - ultimately increasing the bioavailability of said active agent. Conventional pharmaceutical actives commonly do not have a pleasant taste, due to the nature of the therapeutic agents they contain. A palatable fill composition containing honey can further operate as a masking agent for the unpleasant taste of one or more active agents. Capsules comprising honey and other pharmaceutically active compounds, thus provide a new delivery system for enhanced bioavailability for pharmaceuticals and particularly health and nutritional supplements.

As such, a complementary health and/or pharmaceutically active compound may be selected from the group consisting of antitussives, antihistamines, decongestants, alkaloids, laxatives, ion-exchange resins, anti-cholesterolemic, anti-lipid agents,
antiarrhythmics, antipyretics, analgesics, appetite suppressants, expectorants, anti-anxiety agents, anti-ulcer agents, anti-inflammatory substances, coronary dilators, cerebral psychotropics, antimanics, stimulants, gastrointestinal agents, sedatives, anti diarrheal preparations, anti-anginal drugs, vasodialators, anti-hypertensive drugs, vasoconstrictors, migraine treatments, antibiotics, tranquilizers, antipsychotics, antitumor drugs, anticoagulants, antithrombotic drugs, hypnotics, anti-emetics, anti-nauseants, anticonvulsants, neuromuscular drugs, hyper- and hypoglycemic agents, thyroid and anti-thyroid preparation, diuretics, antispasmodics, uterine relaxants. antiobesity drugs, anabolic drugs, erythropoietic drugs, antiasthmatics, cough suppressants, mucolytics, anti-uricemic drugs, vitamins, minerals, herbal extracts, other nutritional supplements and combinations thereof. Examples of suitable nutritional supplements include fish oil, omega 3 oil and bee products, such as Royal Jelly and Propolis; and combinations thereof.

Given the inherent health and medicinal benefits of honey, formulations thereof may in one preferred embodiment, be used for the relief and treatment of symptoms associated with coughs, colds and flu. In a particular preferred embodiment, the encapsulation of a pharmaceutically active compound for the suppression and treatment of coughs, combined with the inherent benefits of honey, may offer significant advantages. Such advantages may include a synergistic function between the pharmaceutical active and the honey, as well as the potential to mask the taste of otherwise unpalatable active ingredients.

In one preferred embodiment said emulsified syrup, particularly emulsified honey, is encapsulated in a gelatin-free shall material. The use of a gelatin free shell material, that is compatible with honey; reduced or eliminated cross-linking and other associated problems. The gelatin free shell material preferably comprises a mixture of iota-carrageenan and at least on modified starch; water, a plasticizer and a buffer. A suitable gelatin free capsule has been previously patented by the Applicant under Australian Patent No. 735699 and Australian Innovation Patent No. 2006100743.

More particularly, the gelatin free material comprises a mixture of iota-carrageenan and at least one modified starch, selected from the group consisting of hydroxypropylated tapioca starch, hydroxypropylated maize starch, acid thinned hydroxypropylated corn starch, potato starch, and pregelatinized modified corn starches. This shell material, and a capsule derived there from, is referred to as *Vegicaps*®*.* Examples of appropriate buffers include sodium and potassium salts. However, other known buffers are also applicable to the present invention.

Furthermore said gelatin free shell material of the present invention can be made of, for example, natural or synthetic polymers, gum and starches. The choice of shell material will depend to a certain extent upon the nature of the ingestible fill material.

In a further preferred embodiment, the shell material may comprise one or more gelatin materials or modified gelatin materials. Examples of a suitable modified gelatin includes, but is not limited to, succinated gelatine. While it is recognized that convention gelatin capsules are incompatible with a syrup or honey fill composition, some special grades of gelatin are formulated purposefully to reduce the tendency of cross-linking by either physical or chemical means. Such modified gelatins may be used in the manufacture of the shell material of the present invention.

Each of the gelatin material, modified gelatin material and gelatin free material may additionally comprise a plasticizer, preferably a polyol. Suitable examples include glycerin, sorbitol, sorbitan and mixtures thereof; however, other known plasticizers would also be appropriate.

Additionally, the formulation and the manufacturing method can be used for other dosage forms including two-piece hard capsules; and soft capsule dosage forms such as swallowable, chewable and squeezable softgels, as presently known in the art.

It is further recognized that the shell material and/or the fill material may further comprise a preservative, and/or at least one pharmaceutically acceptable excipient, and/or additional salts and buffers and colorants, as required to ensure the stability, viability, shelf-life and quality of the resulting product. In another preferred embodiment, the present invention provides a process for producing a capsule for a complementary health or pharmaceutical product comprising:
a) providing a fill material comprising an emulsified syrup, wherein said emulsified syrup comprises 40-50% wt/wt of at least one syrup selected from liquid honey, creamed honey and crystallized honey, 1 to 10% wt/wt of at least one emulsifier, 40 to 55% wt/wt of at least one oil or oil mixture; and (iv) 1 to 5% wt/wt of water;
b) forming a shell material having a chamber; and
c) encapsulating the fill material within the shell material by means of a rotary die process.

Furthermore, the provision of a fill material, as described in step a) of abovementioned process, may further comprise:
i) combining said syrup and said emulsifier;
ii) heating the syrup / emulsifier composition to a temperature in the range 25 to 80°C; preferably 35 to 60°C; more preferably 40 to 45°C;
iii) combining said oil or oil mixture with said syrup-emulsifier composition consecutively in two or more portions;
iv) cooling the composition to ambient temperature, whilst concurrently adding said optional water;
v) optionally deaerating said composition.

The invention will now be described with reference to the following examples. It is to be understood that the examples are provided by way of illustration of the invention and that they are in no way limiting to the scope of the invention.

### Examples

The following three Examples detail possible embodiments and advantages of the present invention. Examples 2 and 3 exemplify the formulation of the required emulsified syrup and the process for preparing capsules thereof. Example 1 provides a comparative example, to further illustrate the advantages of the present invention.

### Comparative Example 1

This formulation is used as a comparison with Example 2 and 3.

Fill formulation:

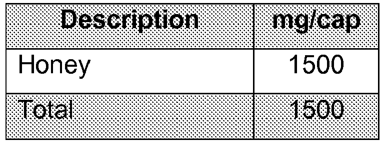

The shell formulation comprises mainly carrageenan, glycerol, water and colorants, as previously described in Australian Patent No. 735699 and Australian Innovation Patent No. 2006100743.

Using a rotary die process, the fill was encapsulated in 20 oblong dies. Heating of the medicine pump was required due to high viscosity of the honey. Many capsules did not form completely on the rotary dies during manufacture, resulting in leakage. After drying, the capsules deformed and sagged around the seams.

### Example 2

Fill formulation:

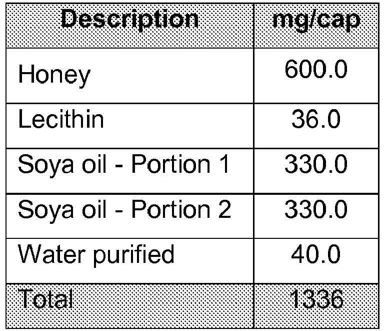

In a vessel fitted with a mechanical stirrer, honey and lecithin were added. Whilst stirring, the mixture was warmed to 40-44°C. Soya oil was added in 2 consecutive portions, whilst concurrently stirring until the mixture was uniform. While cooling, additional water, as required, was added and the mixture stirred thoroughly. The fill was deaerated prior to encapsulation.

The fill was encapsulated in 24 oblong in an opaque shell. The capsule shape after drying was satisfactory.

### Example 3

Fill formulation:

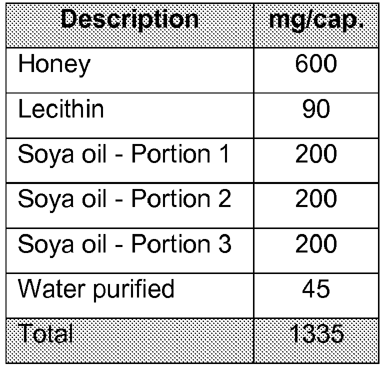

The excipient ratio in this example is different from that of Example 2. The preparation method of the fill is similar to Example 2, except that soya oil was divided into 3 portions for improved emulsification.

As compared to Example 2, the emulsion stability was improved as the result of stepwise input of the emulsifier and/or oil or oil mixture. The ease of encapsulation and the seal strength were increased. Drying time was also reduced.

## Claims

1. A capsule for a complementary health or pharmaceutical product comprising:
(a) a hard or soft shell for encapsulating a fill material; and
(b) a fill material comprising an emulsified syrup wherein the emulsified syrup comprises:
(i) 40 to 50% wt/wt of at least one syrup selected from liquid honey, creamed honey and crystallized honey;
(ii) 1 to 10% wt/wt of at least one emulsifier; and
(iii) 40 to 55% wt/wt of at least one oil or oil mixture; and
(iv) 1 to 5% wt/wt of water.

2. The capsule according to any one of the preceding claims, wherein the emulsifier comprises at least one ionic or a non-ionic edible surfactant, preferably selected from the group comprising lecithin, polyoxyethylene fatty acid esters, glycerides of fatty acids, polyoxyethylene castor oil derivatives and sorbitan esters.

3. The capsule according to any one of the preceding claims, wherein the oil or oil mixture comprises at least one medium or long chain, mono-, di- or tri-glycerides; preferably selected from the group consisting of medium chain triglycerides, soya oil, sunflower oil, safflower oil and rice bran oil.

4. The capsule according to any one of the preceding claims, wherein the total water content of the fill material is less than 12.5% wt/wt.

5. The capsule according to any one of the preceding claims, wherein the fill material further comprises one or more complementary health or pharmaceutically active compounds.

6. The capsule according to claim 5, wherein said complementary health or pharmaceutically active compound is selected from antitussives, antihistamines, decongestants, alkaloids, laxatives, ion-exchange resins, anti-cholesterolemic, anti-lipid agents, antiarrhythmics, antipyretics, analgesics, appetite suppressants, expectorants, anti-anxiety agents, anti-ulcer agents, anti-inflammatory substances, coronary dilators, cerebral psychotropics, antimanics, stimulants, gastrointestinal agents, sedatives, anti-diarrheal preparations, anti-anginal drugs, vasodilators, anti-hypertensive drugs, vasoconstrictors, migraine treatments, antibiotics, tranquilizers, antipsychotics, antitumor drugs, anticoagulants, antithrombotic drugs, hypnotics, anti-emetics, anti-nauseants, anticonvulsants, neuromuscular drugs, hyper- and hypoglycemic agents, thyroid and anti-thyroid preparation, diuretics, antispasmodics, uterine relaxants. antiobesity drugs, anabolic drugs, erythropoietic drugs, antiasthmatics, cough suppressants, mucolytics, anti-uricemic drugs, vitamins, minerals, herbal extracts, fish oil, omega 3 oil, bee products, other nutritional supplements, and combinations thereof.

7. The capsule according to any one of the preceding claims, wherein the shell material and/or fill material further comprises at least one pharmaceutically acceptable excipient.

8. The capsule according to any one of the preceding claims, wherein the shell material is a gelatin material or a modified gelatin material, or combinations thereof; or a gelatin free material.

9. The capsule according to claim 8, wherein the gelatin free material comprises a mixture of iota-carrageenan and at least on modified starch; water, a plasticizer and a buffer, wherein the buffer is preferably a sodium or potassium salt.

10. The capsule claim of claim 9. wherein the shell composition comprising a mixture of iota-carrageenan and at least one modified starch is selected from hydroxypropylated tapioca starch, hydroxypropylated maize starch, acid thinned hydroxypropylated corn starch, potato starch, and pregelatinized modified corn starches.

11. The capsule according to claim 9, wherein said plasticizer is a polyol, preferably selected from glycerin, sorbitol, sorbitan and mixtures thereof.

12. The capsule according to claim 8, wherein the gelatin free material comprises a suitable polymer or gum, or a combination of polymers and/or gums; wherein said polymer or gum is independently selected from natural, semi-synthetic or synthetic polymers and/or gums.

13. A process for producing a capsule according to any one of claims 1-12 for a complementary health or pharmaceutical product comprising:
a) providing a fill material comprising anemulsified syrup, wherein said emulsified syrup comprises:
i. 40 to 50% wt/wt of at least one syrup selected from liquid honey, creamed honey and crystallized honey,
ii. 1 to 10% wt/wt of at least one emulsifier,
iii. 40 to 55% wt/wt of at least one oil or oil mixture; and
(iv) 1 to 5% wt/wt of water;
b) forming a shell material having a chamber; and
c) encapsulating the fill material within the shell material by means of a rotary die process.

14. The process according to claim 13 wherein said gelatin free material comprises a mixture of iota-carrageenan and at least one modified starch; water, a plasticizer and a buffer.

15. A capsule prepared by the process of any one of claims 13 to 14.

## Patentansprüche

1. Kapsel für ein gesundheitliches oder pharmazeutisches Ergänzungsprodukt, umfassend:
(a) eine harte oder weiche Hülle zum Einkapseln eines Füllmaterials, und
(b) ein Füllmaterial, umfassend einen emulgierten Sirup, wobei der emulgierte Sirup aus Folgendem besteht:
(i) 40 bis 50 % Massenanteil mindestens eines Sirups, ausgewählt aus flüssigem Honig, cremigem Honig und kristallisiertem Honig,
(ii) 1 bis 10 % Massenanteil mindestens eines Emulgators, und
(iii) 40 bis 55 % Massenanteil mindestens eines Öls oder eines Ölgemisches, und
(iv) 1 bis 5 % Massenanteil Wasser

2. Kapsel nach einem der vorhergehenden Ansprüche, wobei der Emulgator mindestens ein ionisches oder nichtionisches essbares Tensid umfasst, vorzugsweise ausgewählt aus der Gruppe bestehend aus Lecithin, Polyoxyethylen-Fettsäureestern, Glyceriden von Fettsäuren, Polyoxyethylen-Rizinusöl-Derivaten und Sorbitanestern.

3. Kapsel nach einem der vorhergehenden Ansprüche, wobei das Öl oder Ölgemisch mindestens ein mittel- oder langkettiges Mono-, Di- oder Triglycerid enthält, vorzugsweise ausgewählt aus der Gruppe bestehend aus mittelkettigen Triglyceriden, Sojaöl, Sonnenblumenöl, Distelöl und Reiskleieöl.

4. Kapsel nach einem der vorhergehenden Ansprüche, wobei der Gesamtwassergehalt des Füllmaterials weniger als 12,5 % Massenanteil beträgt.

5. Kapsel nach einem der vorhergehenden Ansprüche, wobei das Füllmaterial ferner eine oder mehrere komplementäre gesundheitlich oder pharmazeutisch aktive Verbindungen umfasst.

6. Kapsel nach Anspruch 5, wobei die komplementäre gesundheitlich oder pharmazeutisch aktive Verbindung ausgewählt ist aus Antitussiva, Antihistaminika, Dekongestionsmitteln, Alkaloiden, Abführmitteln, lonenaustauscherharzen, Anticholesterolämie-, Antilipidmitteln, Antiarrhythmika, Antipyretika, Analgetika, Appetitzüglern, Expectorantien, Anxiolytika, Anti-Ulcus-Mitteln, entzündungshemmenden Substanzen, Koronardilatatoren, zerebralen Psychopharmaka, Antimanika, Stimulanzien, gastrointestinalen Mitteln, Sedativa, Antidiarrhoika, Antianginosa, Vasodilatatoren, Antihypertensiva, Vasokonstriktoren, Migränebehandlungen, Antibiotika, Beruhigungsmitteln, Antipsychotika, Antitumormitteln, Antikoagulantien, antithrombotischen Arzneimitteln, Hypnotika, Antiemetika, Brechreizhemmern, Antikonvulsiva, neuromuskulären Arzneimitteln, Hyper- und Hypoglykämika, Thyroid- und Antithyroidpräparaten, Diuretika, Antispasmodika, Uterusrelaxantien, Antiadiposita, Anabolika, Erythropoetika, Antiasthmatika, Hustenstillern, Mukolytika, antiurikämischen Arzneimitteln, Vitaminen, Mineralstoffen, Kräuterextrakten, Fischöl, Omega-3-Öl, Bienenprodukten, anderen Nahrungsergänzungsmitteln und Kombinationen davon.

7. Kapsel nach einem der vorhergehenden Ansprüche, wobei das Hüllenmaterial und/oder Füllmaterial ferner mindestens einen pharmazeutisch verträglichen Hilfsstoff enthält.

8. Kapsel nach einem der vorhergehenden Ansprüche, wobei das Hüllenmaterial ein Gelatinematerial oder ein modifiziertes Gelatinematerial oder Kombinationen davon oder ein gelatinefreies Material ist.

9. Kapsel nach Anspruch 8, wobei das gelatinefreie Material ein Gemisch aus 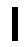 -Carrageen und mindestens einer modifizierten Stärke, Wasser, einem Weichmacher und einem Puffer umfasst, wobei der Puffer vorzugsweise ein Natrium- oder Kaliumsalz ist.

10. Kapsel nach Anspruch 9, wobei die Hüllenzusammensetzung, die ein Gemisch aus 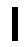 -Carrageen und mindestens einer modifizierten Stärke umfasst, ausgewählt ist aus hydroxypropylierter Tapiokastärke, hydroxypropylierter Maisstärke, säureverdünnter hydroxypropylierter Maisstärke, Kartoffelstärke und vorgelatinierten modifizierten Maisstärken.

11. Kapsel nach Anspruch 9, wobei der Weichmacher ein Polyol ist, vorzugsweise ausgewählt aus Glycerin, Sorbit, Sorbitan und Gemischen davon.

12. Kapsel nach Anspruch 8, wobei das gelatinefreie Material ein geeignetes Polymer oder Gummi oder eine Kombination von Polymeren und/oder Gummis umfasst, wobei das Polymer oder Gummi unabhängig ausgewählt ist aus natürlichen, halbsynthetischen oder synthetischen Polymeren und/oder Gummis.

13. Verfahren zur Herstellung einer Kapsel nach einem der Ansprüche 1 bis 12 für ein gesundheitliches oder pharmazeutisches Ergänzungsprodukt, umfassend:
a) Bereitstellen eines Füllmaterials, umfassend einen emulgierten Sirup, wobei der emulgierte Sirup aus Folgendem besteht:
i. 40 bis 50 % Massenanteil mindestens eines Sirups, ausgewählt aus flüssigem Honig, cremigem Honig und kristallisiertem Honig,
ii. 1 bis 10 % Massenanteil mindestens eines Emulgators, und
iii. 40 bis 55 % Massenanteil mindestens eines Öls oder eines Ölgemisches, und
iv. 1 bis 5 % Massenanteil Wasser
b) Bilden eines Hüllenmaterials mit einer Kammer, und
c) Einkapseln des Füllmaterials innerhalb des Hüllenmaterials mittels eines Rotary-Die-Verfahrens.

14. Verfahren nach Anspruch 13, wobei das gelatinefreie Material ein Gemisch aus 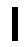 -Carrageen und mindestens einer modifizierter Stärke, Wasser, einem Weichmacher und einem Puffer umfasst.

15. Kapsel, hergestellt nach dem Verfahren eines der Ansprüche 13 bis 14.

## Revendications

1. Capsule pour produit pharmaceutique ou de santé complémentaire comprenant :
(a) une enveloppe dure ou molle pour encapsuler un matériau de remplissage ; et
(b) un matériau de remplissage comprenant un sirop émulsifié où le sirop émulsifié comprend :
(i) de 40 à 50 % en poids d'au moins un sirop choisi parmi du miel liquide, du miel crémeux et du miel cristallisé ;
(ii) de 1 à 10 % en poids d'au moins un émulsifiant ; et
(iii) de 40 à 55 % en poids d'au moins une huile ou mélange d'huiles ; et
(iv) de 1 à 5 % en poids d'eau.

2. Capsule selon l'une quelconque des revendications précédentes, dans laquelle l'émulsifiant comprend au moins un tensioactif comestible ionique ou non ionique, choisi de préférence dans le groupe comprenant lécithine, esters d'acide gras polyéthoxylés, glycérides d'acides gras, dérivés d'huile de ricin polyéthoxylés et esters de sorbitan.

3. Capsule selon l'une quelconque des revendications précédentes, dans laquelle l'huile ou le mélange d'huiles comprend au moins un mono-, di- ou triglycéride à chaîne moyenne ou longue ; de préférence choisi dans le groupe constitué de triglycérides à chaîne moyenne, huile de soja, huile de tournesol, huile de carthame et huile de son de riz.

4. Capsule selon l'une quelconque des revendications précédentes, dans laquelle la teneur totale en eau du matériau de remplissage est inférieure à 12,5 % en poids.

5. Capsule selon l'une quelconque des revendications précédentes, dans laquelle le matériau de remplissage comprend en outre un ou plusieurs composés actifs pharmaceutiquement ou complémentaires de santé.

6. Capsule selon la revendication 5, dans laquelle ledit composé actif pharmaceutiquement ou complémentaire de santé est choisi parmi des antitussifs, antihistaminiques, décongestionnants, alcaloïdes, laxatifs, résines échangeuses d'ion, anticholestérolémiants, agents anti-lipidiques, agents anti-arythmie, antipyrétiques, analgésiques, coupe-faim, expectorants, anxiolytiques, agents anti-ulcéreux, substances anti-inflammatoires, dilatateurs coronariens, psychotropes cérébraux, agents contre la manie, stimulants, agents gastro-intestinaux, sédatifs, préparations anti-diarrhéiques, médicaments anti-angineux, vasodilatateurs, médicaments anti-hypertenseurs, vasoconstricteurs, traitements contre la migraine, antibiotiques, tranquillisants, antipsychotiques, médicaments antitumoraux, anticoagulants, médicaments antithrombiques, hypnotiques, anti-émétiques, anti-nauséeux, anticonvulsants, médicaments neuromusculaires, agents hyper- et hypoglycémiants, préparation thyroïdienne et anti-thyroïdienne, diurétiques, antispasmodiques, relaxants utérins, médicaments contre l'obésité, médicaments anabolisants, médicaments érythropoiétiques, antiasthmatiques, agents contre la toux, mucolytiques, médicaments hypo-uricémiants, vitamines, minéraux, extraits végétaux, huile de poisson, huile aux oméga 3, produits apicoles, autres compléments nutritionnels, et des combinaisons de ceux-ci.

7. Capsule selon l'une quelconque des revendications précédentes, dans laquelle le matériau d'enveloppe et /ou le matériau de remplissage comprend en outre au moins un excipient pharmaceutiquement acceptable.

8. Capsule selon l'une quelconque des revendications précédentes, dans laquelle le matériau d'enveloppe est un matériau à la gélatine ou un matériau à la gélatine modifiée, ou des combinaisons de ceux-ci ; ou un matériau exempt de gélatine.

9. Capsule selon la revendication 8, dans laquelle le matériau exempt de gélatine comprend un mélange de iota-carraghénane et au moins un amidon modifié ; de l'eau, un plastifiant et un tampon, le tampon étant de préférence un sel de sodium ou de potassium.

10. Capsule selon la revendication 9, dans laquelle la composition de l'enveloppe comprenant un mélange de iota-carraghénane et au moins un amidon modifié est choisie parmi de l'amidon de tapioca hydroxypropylé, de l'amidon de maïs hydroxypropylé, de l'amidon de maïs hydroxypropylé dilué à l'acide, de l'amidon de pomme de terre et des amidons de maïs modifiés prégélatinisés.

11. Capsule selon la revendication 9, dans laquelle ledit plastifiant est un polyol, choisi de préférence parmi la glycérine, le sorbitol, le sorbitan et des mélanges de ceux-ci.

12. Capsule selon la revendication 8, dans laquelle le matériau exempt de gélatine comprend un polymère ou une gomme approprié, ou une combinaison de polymères et/ou de gommes ; ledit polymère ou ladite gomme étant choisi(e) indépendamment parmi des polymères et/ou des gommes semi-synthétiques ou synthétiques.

13. Procédé de production de capsule selon l'une quelconque des revendications 1 à 12 pour un produit pharmaceutique ou complémentaire de santé comprenant :
a) la fourniture d'un matériau de remplissage comprenant un sirop émulsifié, ledit sirop émulsifié comprenant :
i. de 40 à 50 % en poids d'au moins un sirop choisi parmi du miel liquide, du miel crémeux et du miel cristallisé,
ii. de 1 à 10 % en poids d'au moins un émulsifiant
iii. de 40 à 55 % en poids d'au moins une huile ou mélange d'huiles ; et
(iv) de 1 à 5 % en poids d'eau ;
b) la formation d'un matériau d'enveloppe ayant une chambre ; et
c) l'encapsulation du matériau de remplissage à l'intérieur du matériau d'enveloppe au moyen d'un procédé à moule rotatoire.

14. Procédé selon la revendication 13 dans lequel ledit matériau exempt de gélatine comprend un mélange de iota-carraghénane et au moins un amidon modifié ; de l'eau, un plastifiant et un tampon.

15. Capsule préparée par le procédé selon l'une quelconque des revendications 13 à 14.
